# EUROPEAN PATENT APPLICATION

(11) **EP 3 741 334 A1**
(43) Date of publication of application: **25.11.2020**
(21) Application number: 19176153.5
(22) Date of filing: 23.05.2019
(51) Int. Cl.: A61F 11/08, H04R 5/00

(54) **ADAPTIVE EAR PROTECTION SYSTEM FOR A MEDICAL IMAGING DEVICE**

(71) Applicant: Koninklijke Philips N.V., 5656 AG Eindhoven (NL)
(72) Inventor: WEISS, Steffen, 5656 AE Eindhoven (NL); JOHNSON, Mark Thomas, 5656 AE Eindhoven (NL)
(74) Representative: de Haan, Poul Erik

(57) **Abstract**

The present invention relates to an adaptive ear protection system (10) for a medical imaging device (200). The adaptive ear protection system comprises a pair of ear muffs (20), an actuation system (30), at least one sensor system (40), and a processing unit (50). The pair of ear muffs are configured to fit around the ears of a patient. The actuation system is configured to adjust the fit of the pair of ear muffs around the ears of the patient. The at least one sensor system is configured to determine a measurement of an effectiveness of fit of the pair of ear muffs around the ears of the patient. The processing unit is configured to control the actuation system to adjust the fit of the pair of ear muffs around the ears of the patient, the control comprising utilization of the measurement of effectiveness of fit of the pair of ear muffs around the ears of the patient.

## Description

### FIELD OF THE INVENTION

The present invention relates to adaptive ear protection systems for a medical imaging device, and methods of adaptive ear protection for a medical imaging device, as well as to a computer program element and a computer readable medium.

### BACKGROUND OF THE INVENTION

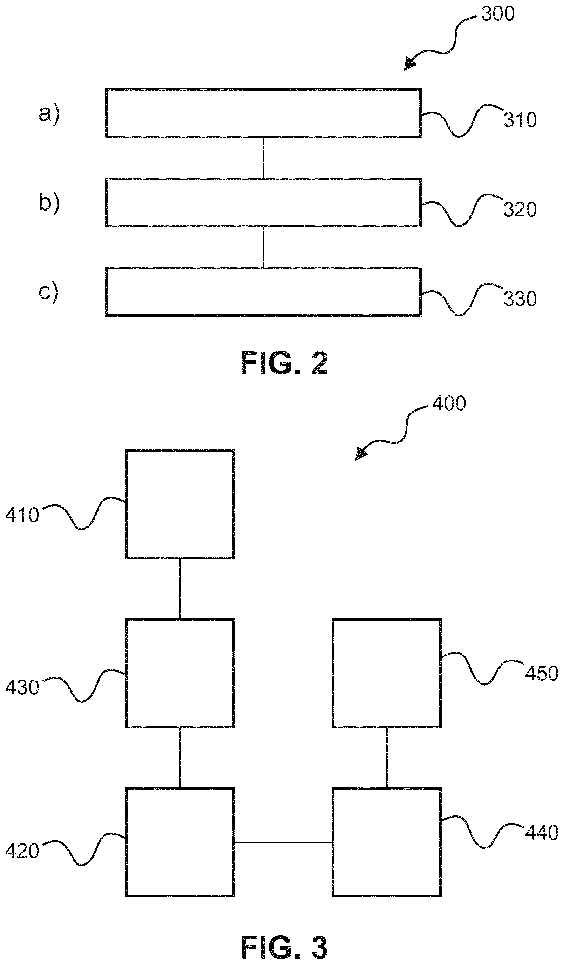

Patients undergoing Magnetic Resonance Imaging (MRI) are routinely subjected to high sound pressure levels, averaging around 95-105 dB, and for some MR pulse sequences this can increase up to 130 dB and even beyond (Moelker A et al. Importance of Bone-Conducted Sound Transmission on Patient Hearing in the MR Scanner. J Magn Reson Imag 2005 ;22:163-169). Therefore, the wearing of ear protection is mandatory during MR exams, and different sorts of ear protection are used, mostly in the form of ear muffs and in-ear foam plugs as shown in Fig. 3. Ear muffs are more widely used, because they can be applied more easily and quickly, and because ear plugs are inconvenient for many patients and may not be applied properly by children, elderly, or demented patients. The efficiency of protection depends critically on their correct application, but patients are often not used to wearing such ear protection, or are incapable of applying them properly. Consequently, studies have shown that the actual attenuation of sound for patients is 5dB to 25dB lower than the rated attenuation when devices are applied by normal users (Trompette N, Kusy A. Suitability of Commercially Available Systems for Individual Fit Tests of Hearing Protectors. Internoise 2013, Innsbruck). The reason for this difference is mainly incorrect application by the end user. This results in a general problem in MR, because most patients are not used to apply ear protection. Patients lack experience of what the correct level of attenuation should be and are not aware that they may be experiencing excessive noise levels, and there are no objective measures to check proper attenuation in-situ. Thus, there is no objective measure to check whether ear muffs or plugs have been applied properly, rather sometimes a subjective check is performed in which the radiographer asks the patient during patient preparation, whether the devices fit tightly. Ear muffs have to be applied such that there is no break or opening in the seal around the ear. On the other hand, the pressure with which the muffs are compressed to the head should remain low. Otherwise, patients can become uncomfortable during longer scans and may move to adjust the device for a more convenient fit, or even cause scan interrupts by calling for assistance. Standard ear muffs used in MR have a one-fits-all size. In a few cases a second one-fits-all version for paediatric patients is used. A head clamp provides the force for compressing the muffs to the head. The clamp comprises a spring mechanism such that the applied compression depends on the size of the head of the patient. This may result in a too high a pressure in big patients and in a too low a pressure for small patients in comparison to the minimum reasonable pressure required for tight fit.

### SUMMARY OF THE INVENTION

It would be advantageous to have improved means of providing ear protection during medical examination by a medical imaging device, such as a MR imaging device. The object of the present invention is solved with the subject matter of the independent claims, wherein further embodiments are incorporated in the dependent claims. It should be noted that the following described aspects and examples of the invention apply also to the adaptive ear protection systems for a medical imaging device, the methods of adaptive ear protection for a medical imaging device, as well as to the computer program element and a computer readable medium.

According to a first aspect, there is provided an adaptive ear protection system for a medical imaging device. The adaptive ear protection system comprises:
- a pair of ear muffs;
- an actuation system;
- at least one sensor system; and
- a processing unit;

The pair of ear muffs are configured to fit around the ears of a patient. The actuation system is configured to adjust the fit of the pair of ear muffs around the ears of the patient. The at least one sensor system is configured to determine a measurement of an effectiveness of fit of the pair of ear muffs around the ears of the patient. The processing unit is configured to control the actuation system to adjust the fit of the pair of ear muffs around the ears of the patient, the control comprising utilization of the measurement of effectiveness of fit of the pair of ear muffs around the ears of the patient.

In this way an ear protection system is provided to protect a patient during a procedure such as Magnetic Resonance imaging MRI, where noise levels typically are around 95-105dB and can be 130dB and above. The wearing of ear protectors is mandatory for an MRI examination, however patients are generally not used to wearing such protection and consequently may not wear the protectors appropriately for the required level of protection, a situation exacerbated for children, elderly and patients with dementia. The system addresses this through monitoring the effectiveness of the protection being provided by the ear protectors, and provides an indicating signal useable to enable an actuation system to adjust the compression of the ear protectors to the head of the patient. In this way, the system enables the actuation system to adjust the compression until a correctly tight seal is provided around the ear protectors and thus sufficient attenuation of sound is obtained. Also, because the correct level of compression is enabled to be provided, ear protectors that mitigation of the provision of ear protectors that are too tightly compressed against the head is provided. In other words, the system results in generally lower levels of compression, increasing the comfort to the patient during long scans. The actuation system can be a pneumatic system, or a hydraulic system for example, or comprise electromagnetic actuators, such as piezo-electric elements or responsive thin film materials or movement actuators utilizing an induction coil in a similar manner to a loudspeaker coil.

Thus, an objective measurement is provided of the attenuation be provided for ear protection, enabling remedial action to be taken if the incorrect level of protection is currently being provided.

In an example, the sensor system is configured to provide the at least one measurement of effectiveness of fit of the pair of ear muffs around the ears of the patient to the patient. The processing unit is configured to receive an ear muff fit instruction. The processing unit is configured to control the actuation system to adjust the fit of the pair of ear muffs around the ears of the patient comprising utilization of the ear muff fit instruction.

Thus, the patient can be asked to adjust the level of compression if the seal is not tight enough, thereby putting the patient in the control loop and helping to reduce any anxiety they may have.

In an example, the at least one sensor system comprises at least one microphone. The measurement of effectiveness of fit of the pair of ear muffs around the ears of the patient comprises at least one measured sound level.

In an example, the at least one microphone is external to the pair of ear muffs. In an example, the at least one sensor system comprises a pair of air tubes. A first air tube of the pair of air tubes connects an inner chamber of a first ear muff that is configured to surround a first ear of the patient to the at least one microphone. A second air tube of the pair of air tubes connects an inner chamber of a second ear muff that is configured to surround a second ear of the patient to the at least one microphone.

In other words, air tubes connect the inner portion of the ear muffs, to detect the level of noise to which the patient's ears are being subjected, to microphones that are external to the ear muffs, for example integrated into an in-room operating console of an MRI system, or integrated with the MRI head coil, or integrated with the patient support for example. In this manner, the wired microphones are at a safe distance from the patient and are safely outside of the imaging region of the MRI system, whilst passive air tubes are in that region transferring the level of sound at the patient's ears to these external microphones, where that level of sound indicates the effectiveness of fit of the ear protectors. Thus, an automated ear protection system can be provided, where the actual level of sound the patient is being subjected to is monitored in order to automatically adjust the compression of ear protectors until the required level of attenuation is provided, and where the patient and/or device operator can be involved in this process through being able to manually change the level of compression for example.

Thus, the sound level is measured after attenuation by additional microphones. However, rather than the integration of such microphones into the ear muffs, which is prohibitive for RF safety reasons, air tubes are used for connection between the muffs and microphones that are external to the ear muffs, for example integrated in the MR head coil.

In an example, the at least one sensor system comprises at least one photosensitive device. The at least one photosensitive device is configured to detect light leakage past a seal around an inner chamber of a first ear muff that is configured to surround a first ear of the patient. The at least one photosensitive device is configured to detect light leakage past a seal around an inner chamber of a second ear muff that is configured to surround a second ear of the patient. The measurement of effectiveness of fit of the pair of ear muffs around the ears of the patient comprises at least one measured light level.

In other words, an optical waveguide such as an optical fibre can be positioned at the seal for one ear muff and an optical fibre can be positioned at the seal for the second ear muff, with both running to a photodiode and light leakage can be detected past the seal to determine an effectiveness of fit. Or two photodiodes can be used, where each is ear muff has a photodiode associated with it.

In an example, the at least one sensor system comprises a pair of photosensitive devices. A first photosensitive device of the pair of photosensitive devices is located with respect to a seal around an inner chamber of a first ear muff that is configured to surround a first ear of the patient. The first photosensitive device is located to detect light leakage past the seal. A second photosensitive device of the pair of photosensitive devices is located with respect to a seal around an inner chamber of a second ear muff that is configured to surround a second ear of the patient. The second photosensitive device is located to detect light leakage past the seal. The measurement of effectiveness of fit of the pair of ear muffs around the ears of the patient comprises at least one measured light level.

In other words, a "proxy" noise detection system is provided to determine the effectiveness of fit of the ear protectors, where a light leakage approach is used. Thus, in an example for a non-transparent pair of ear muffs, a photosensitive device such as a photodiode is used inside the ear muff, and any light leaking into the ear muff is detected by the photodiode and is a direct indicator of the effectiveness of the fit of the ear muffs, and a level of compression can be applied to provide the required level of seal around the ear muffs to provide the required level of ear protection. Or, in another example a light source is provided inside the ear muffs and a photosensitive device is positioned outside the ear muff, and any light leaking from inside to outside is a direct indicator of the effectiveness of the fit of the ear muffs, and a level of compression can be applied to provide the required level of seal around the ear muffs to provide the required level of ear protection. Thus, in this example the system becomes independent of the light level, and can operate when there is no ambient lighting. Additionally, in an example the light source can be infrared in order to differentiate from ambient lighting and/or be modulated in a known way in order that the light from the photodiode can be detected even when there is ambient lighting

Thus, an automated ear protection system can be provided, where a proxy indicator is used to measure the level of sound the patient is being subjected to and directly measure an effectiveness of fit of the ear muffs based on light monitoring in order to automatically adjust the compression of ear protectors until the required level of attenuation is provided, and where the patient and/or device operator can be involved in this process through being able to manually change the level of compression for example.

In an example, the at least one sensor system comprises a pair of electrodes. A first electrode of the pair of electrodes is located at a periphery of a first ear muff such that when the first ear muff is positioned to surround a first ear of the patient the first electrode is configured to contact and/or be adjacent to skin of the patient. A second electrode of the pair of electrodes is located at a periphery of a second ear muff such that when the second ear muff is positioned to surround a second ear of the patient the second electrode is configured to contact and/or be adjacent to skin of the patient. The measurement of effectiveness of fit of the pair of ear muffs around the ears of the patient comprises at least one impedance, conductance, or capacitance level.

In other words, a "proxy" noise detection system is provided to determine the effectiveness of fit of the ear protectors, where a number of different electrical approaches can be used. Thus, electrical monitoring using appropriate sensors on the seal position of the ear muffs is used to measure impedance, conductance or capacitance, and is a direct indicator of the effectiveness of the fit of the ear muffs, a proxy indicator of the noise attenuation being provided, and a level of compression can be applied to provide the required level of seal around the ear muffs to provide the required level of ear protection. Thus, an automated ear protection system can be provided, where a proxy indicator is used to measure the level of sound the patient is being subjected to and directly measure an effectiveness of fit of the ear muffs based on electrical signals in order to automatically adjust the compression of ear protectors until the required level of attenuation is provided, and where the patient and/or device operator can be involved in this process through being able to manually change the level of compression for example.

In other words, a measurement with one electrode associated with an ear muff can be used to determine how tight that ear muff is around the ear. The electrode associated with the other ear muff can similarly be used to determine how tight that ear muff is around the ear.

Thus, one electrode can be used to determine an effectiveness of fit, for example against ground level. However, in an example two or more electrodes can be used around the periphery or seal of each ear muff, and impedance, conductance or capacitance can be measured between adjacent electrodes to determine an effectiveness or tightness of fit. Indeed, electrodes can be spaced all around the seal of each ear muff and when in contact with the skin of the patient impedance, conductance or capacitance can be measured between adjacent electrodes to determine an effectiveness or tightness of fit. This can be used to detect where exactly around the seal there is some leakage (e.g. at 6 o'clock) and to indicate this to the patient or staff in form of a command to adapt the device at that location.

In an example, the at least one sensor system comprises a pair of gas pressure sensors. A first gas pressure sensor of the pair of gas pressure sensors is located within an inner chamber of a first ear muff that is configured to surround a first ear of the patient. A second gas pressure sensor of the pair of gas pressure sensors is located within an inner chamber of a second ear muff that is configured to surround a second ear of the patient. The measurement of effectiveness of fit of the pair of ear muffs around the ears of the patient comprises at least one measured gas pressure level.

In other words, a "proxy" noise detection system is provided to determine the effectiveness of fit of the ear protectors, where a slight positive or negative pressure differential between the inside of the ear muff and the outside can be used to provide a direct indicator of the effectiveness of the fit of the ear muffs, and a proxy indicator of the noise attenuation is provided, and a level of compression can be applied to provide the required level of seal around the ear muffs to provide the required level of ear protection. Thus, an automated ear protection system can be provided, where a proxy indicator is used to measure the level of sound the patient is being subjected to and directly measure an effectiveness of fit of the ear muffs based on pressure signals in order to automatically adjust the compression of ear protectors until the required level of attenuation is provided, and where the patient and/or device operator can be involved in this process through being able to manually change the level of compression for example.

In an example, the at least one sensor system comprises a pair of gas injectors and a pair of gas sensors. A first gas injector and a first gas sensor is located within an inner chamber of a first ear muff that is configured to surround a first ear of the patient. The first gas injector is configured to inject a gas other than air into the inner chamber. A second gas injector and a second gas sensor is located within an inner chamber of a second ear muff that is configured to surround a second ear of the patient. The second gas injector is configured to inject a gas other than air into the inner chamber. The measurement of effectiveness of fit of the pair of ear muffs around the ears of the patient comprises at least one measured gas composition level.

In other words, a "proxy" noise detection system is provided to determine the effectiveness of fit of the ear protectors, where a gas composition or a change in gas composition inside of the ear muff can be used to provide a direct indicator of the effectiveness of the fit of the ear muffs because this can indicate that gas from inside is leaking to the outside and air from the outside is leaking into the air muff changing the gas composition and indicating that the ear muffs are not correctly sealed to the patient's head, and a proxy indicator of the noise attenuation is provided. A level of compression can then be applied to provide the required level of seal around the ear muffs to provide the required level of ear protection. Thus, an automated ear protection system can be provided, where a proxy indicator is used to measure the level of sound the patient is being subjected to and directly measure an effectiveness of fit of the ear muffs based on gas composition in order to automatically adjust the compression of ear protectors until the required level of attenuation is provided, and where the patient and/or device operator can be involved in this process through being able to manually change the level of compression for example.

In an example, utilization of the measurement of effectiveness of fit of the pair of ear muffs around the ears of the patient comprises a comparison of the measurement of effectiveness of fit of the pair of ear muffs around the ears of the patient with a threshold level, and wherein the processing unit is configured to control the actuation system to increase a level of compression of the fit of the pair of ear muffs around the ears of the patient on the basis of the comparison.

In this way, the ear muffs can be compressed to exactly the right level to provide the required attenuation, and not be compressed to tightly against the patient's head.

In an example, the processing unit is configured to receive scan information from the medical imaging device. The processing unit is configured to control the actuation system to adjust the fit of the pair of ear muffs around the ears of the patient, the control comprising utilization of the scan information.

In this manner, pre-emptive levels of compression can be provided, for example when a next stage of scanning will be especially loud, signals from the scanner can be used to provide extra compression and a greater degree of attenuation is preparation for the loud sounds, thereby protecting the patient. When, the scanner is entering into a normal or quiescent mode, the ear muffs can be again correctly compressed in a not too tight manner, but also providing a safe level of sound attenuation, thereby providing for maximised comfort, with maximised safety for patients.

According to a second aspect, there is provided an adaptive ear protection system for a medical imaging device, the adaptive ear protection system comprising:
- a pair of ear plugs;
- at least one microphone;
- a pair of air tubes;
- a processing unit; and
- an output unit.

The pair of ear plugs are configured to fit into the ears of a patient. The at least one microphone is external to the ear plugs. A first air tube of the pair of air tubes extends through a first ear plug of the pair of ear plugs and connects to the at least one microphone. A second air tube of the pair of air tubes extends through a second ear plug of the pair of ear plugs and connects to the at least one microphone. The at least one microphone is configured to provide at least one signal indicative of a measured sound level to the processing unit. The processing unit is configured to determine an effectiveness of fit of the pair of ear plugs in the ears of the patient, the determination comprising utilization of the at least one signal indicative of a measured sound level. The output unit is configured to output an indication that the ear plug fit is required to be adapted on the basis of the determined effectiveness of fit of the pair of ear plugs.

In this manner, warnings can be issued if it is determined that the patient is experiencing too high a sound level, and an operator/patient can adapt the ear plugs, for example by seating them more firmly within the patient's ears, or an automatic scan abort can be initiated.

According to a third aspect, there is provided a method of adaptive ear protection for a medical imaging device, the method comprising:
a) fitting a pair of ear muffs the ears of a patient, wherein an actuation system is configured to adjust the fit of the pair of ear muffs around the ears of the patient;
b) determining by at least one sensor system a measurement of an effectiveness of fit of the pair of ear muffs around the ears of the patient; and
c) controlling by a processing unit the actuation system to adjust the fit of the pair of ear muffs around the ears of the patient, the controlling comprising utilizing the measurement of effectiveness of fit of the pair of ear muffs around the ears of the patient.

According to a fourth aspect, there is provided a method of adaptive ear protection system for a medical imaging device, the method comprising:
al) fitting a pair of ear plugs into the ears of a patient, wherein at least one microphone is external to the ear plugs, and wherein a first air tube of a pair of air tubes extends through a first ear plug of the pair of ear plugs and connects to the at least one microphone, and wherein a second air tube of the pair of air tubes extends through a second ear plug of the pair of ear plugs and connects to the at least one microphone;
b1) providing by the at least one microphone at least one signal indicative of a measured sound level to a processing unit;
c1) determining by the processing unit an effectiveness of fit of the pair of ear plugs in the ears of the patient, the determining comprising utilizing the at least one signal indicative of a measured sound level; and
d1) outputting by an output unit an indication that the ear plug fit is required to be adapted on the basis of the determined effectiveness of fit of the pair of ear plugs.

According to another aspect, there is provided a computer program element controlling one or more of the systems as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an adaptive ear protection system for a medical imaging device;
Fig. 2 shows a method of adaptive ear protection for a medical imaging device;
Fig. 3 shows a schematic set up of an example of an adaptive ear protection system for a medical imaging device;
Fig. 4 shows a method of adaptive ear protection for a medical imaging device;
Fig. 5 shows examples of standard ear muffs and in-ear foam plugs commonly used for sound protection during MR exams or scans;
Fig. 6 shows an example of a MR head-coil consisting of a base part and top part, also showing the base part alone; and
Fig. 7 shows examples of ear muffs of an adaptive ear protection system with a pneumatic system fed by pressurized air, showing in the example with two ear muffs an example of how increased air pressure can be used to decrease compression around the ears and showing in the example with one ear muff an example of how increased air pressure can be used to increase compression around the ear.

### DETAILED DESCRIPTION OF EMBODIMENTS

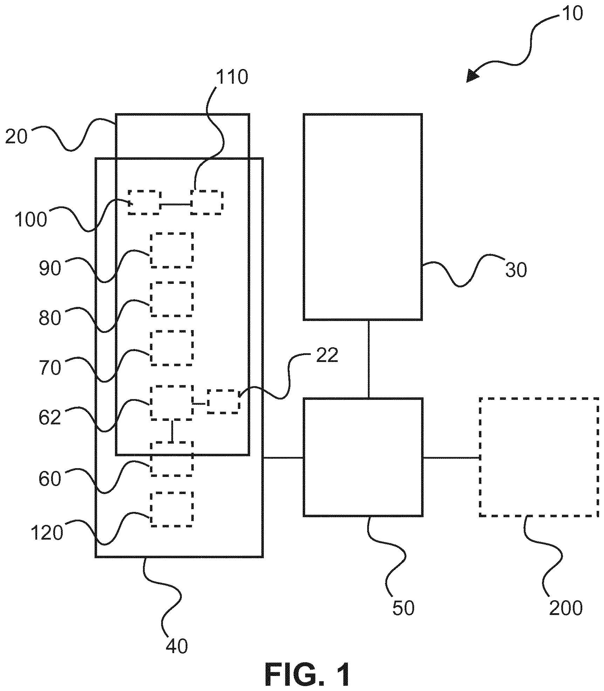

Fig. 1 shows an example of an adaptive ear protection system 10 for a medical imaging device 200. The adaptive ear protection system comprises a pair of ear muffs 20, an actuation system 30, at least one sensor system 40, and a processing unit 50. The pair of ear muffs are configured to fit around the ears of a patient. The actuation system is configured to adjust the fit of the pair of ear muffs around the ears of the patient. The at least one sensor system is configured to determine a measurement of an effectiveness of fit of the pair of ear muffs around the ears of the patient. The processing unit is configured to control the actuation system to adjust the fit of the pair of ear muffs around the ears of the patient. The control comprises utilization of the measurement of effectiveness of fit of the pair of ear muffs around the ears of the patient.

In an example, the actuation system comprises at least one piston 32.

In an example, the actuation system is a pneumatic system.

In an example, the actuation system is a hydraulic system.

In an example, the actuation system is a piezoelectric system.

In an example, the actuation system is a thin film responsive material system.

In an example, the actuation system is an electromagnetic system.

In an example, the processing unit is configured to issue a command to adapt the fit of the ear muffs. Thus, the system can automatically indicate to the operator and/or patient that the ear muffs need to be adjusted.

According to an example, the sensor system is configured to provide the at least one measurement of effectiveness of fit of the pair of ear muffs around the ears of the patient to the patient. The processing unit is configured to receive an ear muff fit instruction. The processing unit is configured to control the actuation system to adjust the fit of the pair of ear muffs around the ears of the patient comprising utilization of the ear muff fit instruction.

In an example, the muff fit instruction is received from the operator. In an example, the muff fit instruction is received from the patient - for example, the patient can indicate that the fit is not tight enough.

In this manner, the medical operative can ensure that the correct level of ear protection is provided, thereby facilitating the provision of the required level of ear protection attenuation to children, elderly and patients with dementia. Also, in this way the medical operative is also notified if a problem has occurred, where for example the patient is pulling away the ear protectors from their head and can halt the scan to protect the patient.

According to an example the at least one sensor system comprises at least one microphone 60. The measurement of effectiveness of fit of the pair of ear muffs around the ears of the patient comprises at least one measured sound level.

According to an example the at least one microphone is external to the pair of ear muffs.

According to an example the at least one sensor system comprises a pair of air tubes 62. A first air tube of the pair of air tubes connects an inner chamber of a first ear muff that is configured to surround a first ear of the patient to the at least one microphone. A second air tube of the pair of air tubes connects an inner chamber of a second ear muff that is configured to surround a second ear of the patient to the at least one microphone.

In an example, the first and second air tubes extend through two ear plugs 22. One ear plug is associated with one ear muff and can be pushed into one ear of the patient and the second ear plug is associated with the other ear muff and can be pushed into the other ear of the patient. In this way the sound level that is measured is that directly at the ear and the ear plugs provide for an additional level of protection.

According to an example, the at least one sensor system comprises at least one photosensitive device 70. The at least one photosensitive device is configured to detect light leakage past a seal around an inner chamber of a first ear muff that is configured to surround a first ear of the patient. The at least one photosensitive device is configured to detect light leakage past a seal around an inner chamber of a second ear muff that is configured to surround a second ear of the patient. The measurement of effectiveness of fit of the pair of ear muffs around the ears of the patient comprises at least one measured light level.

In an example, the sensor system comprises a pair of optical waveguides. A first optical waveguide of the pair of optical waveguides has one end located with respect to the seal around the inner chamber of the first ear muff and a second end located to supply radiation to a photosensitive device. The first optical waveguide in combination with the photosensitive device is configured to detect light leakage past the seal. A second optical waveguide of the pair of optical waveguides has one end located with respect to the seal around the inner chamber of the second ear muff and a second end located to supply radiation to the photosensitive device. The first optical waveguide in combination with the photosensitive device is configured to detect light leakage past the seal. The measurement of effectiveness of fit of the pair of ear muffs around the ears of the patient comprises at least one measured light level.

Thus, in this manner a single photosensitive device such as a photodiode, used in combination with appropriately positioned waveguides such as optical fibres can be used to determine the effectiveness of fit of the ear muffs.

In an example the at least one sensor system comprises a pair of photosensitive devices 70. A first photosensitive device of the pair of photosensitive devices is located with respect to a seal around an inner chamber of a first ear muff that is configured to surround a first ear of the patient. The first photosensitive device is located to detect light leakage past the seal. A second photosensitive device of the pair of photosensitive devices is located with respect to a seal around an inner chamber of a second ear muff that is configured to surround a second ear of the patient. The second photosensitive device is located to detect light leakage past the seal. The measurement of effectiveness of fit of the pair of ear muffs around the ears of the patient comprises at least one measured light level.

According to an example the at least one sensor system comprises a pair of electrodes 80. A first electrode of the pair of electrodes is located at a periphery of a first ear muff such that when the first ear muff is positioned to surround a first ear of the patient the first electrode is configured to contact and/or be adjacent to skin of the patient. A second electrode of the pair of electrodes is located at a periphery of a second ear muff such that when the second ear muff is positioned to surround a second ear of the patient the second electrode is configured to contact and/or be adjacent to skin of the patient. The measurement of effectiveness of fit of the pair of ear muffs around the ears of the patient comprises at least one impedance, conductance, or capacitance level.

In an example two or more electrodes can be used around the periphery or seal of each ear muff, and impedance, conductance or capacitance can be measured between adjacent electrodes to determine an effectiveness or tightness of fit. This can be used to detect where exactly around the seal there is some leakage (e.g. at 6 o'clock) and to indicate this to the patient or staff in form of a command to adapt the device at that location.

According to an example the at least one sensor system comprises a pair of gas pressure sensors 90. A first gas pressure sensor of the pair of gas pressure sensors is located within an inner chamber of a first ear muff that is configured to surround a first ear of the patient. A second gas pressure sensor of the pair of gas pressure sensors is located within an inner chamber of a second ear muff that is configured to surround a second ear of the patient. The measurement of effectiveness of fit of the pair of ear muffs around the ears of the patient comprises at least one measured gas pressure level.

According to an example the at least one sensor system comprises a pair of gas injectors 100 and a pair of gas sensors 110. A first gas injector and a first gas sensor is located within an inner chamber of a first ear muff that is configured to surround a first ear of the patient. The first gas injector is configured to inject a gas other than air into the inner chamber. A second gas injector and a second gas sensor is located within an inner chamber of a second ear muff that is configured to surround a second ear of the patient. The second gas injector is configured to inject a gas other than air into the inner chamber. The measurement of effectiveness of fit of the pair of ear muffs around the ears of the patient comprises at least one measured gas composition level.

According to an example utilization of the measurement of effectiveness of fit of the pair of ear muffs around the ears of the patient comprises a comparison of the measurement of effectiveness of fit of the pair of ear muffs around the ears of the patient with a threshold level. The processing unit is configured to control the actuation system to increase a level of compression of the fit of the pair of ear muffs around the ears of the patient on the basis of the comparison.

In an example, the at least one sensor system comprises highly resistive wiring 120.

Thus, wiring is provided that can sit for example in the MRI machine and be RF compatible.

According to an example the processing unit is configured to receive scan information from the medical imaging device 200). The processing unit is configured to control the actuation system to adjust the fit of the pair of ear muffs around the ears of the patient, the control comprising utilization of the scan information.

In an example, the adaptive ear protection system is configured to operate with a magnetic resonance imaging device, scanner or imager.

Fig. 2 shows a method 300 of adaptive ear protection for a medical imaging device in its basic steps. The method 300 comprises:
- in a fitting step 310, also referred to as step a), fitting a pair of ear muffs the ears of a patient, wherein a actuation system is configured to adjust the fit of the pair of ear muffs around the ears of the patient;
- in a determining step 320, also referred to as step b), determining by at least one sensor system a measurement of an effectiveness of fit of the pair of ear muffs around the ears of the patient; and
- in a controlling step 330, also referred to as step c), controlling by a processing unit the actuation system to adjust the fit of the pair of ear muffs around the ears of the patient, the controlling comprising utilizing the measurement of effectiveness of fit of the pair of ear muffs around the ears of the patient.

In an example, step b) comprises providing the at least one measurement of effectiveness of fit of the pair of ear muffs around the ears of the patient to the patient. Step c) can then comprise receiving an ear muff fit instruction from the patient and utilizing the ear muff fit instruction from the patient to control the actuation system to adjust the fit of the pair of ear muffs around the ears of the patient.

In an example, step b) comprises providing the at least one measurement of effectiveness of fit of the pair of ear muffs around the ears of the patient to an operator of the medical imaging device. Step c) can then comprise receiving an ear muff fit instruction from the operator of the medical imaging device and utilizing the ear muff fit instruction from the operator of the medical imaging device to control the actuation system to adjust the fit of the pair of ear muffs around the ears of the patient.

In an example, the at least one sensor system comprises at least one microphone; and in step b) the measurement of effectiveness of fit of the pair of ear muffs around the ears of the patient comprises at least one measured sound level.

In an example, the at least one microphone is external to the pair of ear muffs.

In an example, the method comprises connecting a first air tube of a pair of air tubes from an inner chamber of a first ear muff that is configured to surround a first ear of the patient to at least one microphone and connecting a second air tube of the pair of air tubes from an inner chamber of a second ear muff that is configured to surround a second ear of the patient to the at least one microphone.

In an example, the at least one sensor system comprises a pair of photosensitive devices and the method comprises locating a first photosensitive device of the pair of photosensitive devices with respect to a seal around an inner chamber of a first ear muff that is configured to surround a first ear of the patient. The first photosensitive device is located to detect light leakage past the seal. The method also comprises locating a second photosensitive device of the pair of photosensitive devices with respect to a seal around an inner chamber of a second ear muff that is configured to surround a second ear of the patient. The second photosensitive device is located to detect light leakage past the seal. In step b) the measurement of effectiveness of fit of the pair of ear muffs around the ears of the patient can comprise utilizing at least one measured light level.

In an example, the at least one sensor system comprises a pair of electrodes, and the method comprises locating a first electrode of the pair of electrodes at a periphery of a first ear muff such that when the first ear muff is positioned to surround a first ear of the patient the first electrode is configured to contact and/or be adjacent to skin of the patient. The method also comprises locating a second electrode of the pair of electrodes at a periphery of a second ear muff such that when the second ear muff is positioned to surround a second ear of the patient the second electrode is configured to contact and/or be adjacent to skin of the patient. In step b) the measurement of effectiveness of fit of the pair of ear muffs around the ears of the patient can comprise utilization of at least one impedance, conductance, or capacitance level.

In an example, the at least one sensor system comprises a pair of gas pressure sensors, and the method comprises locating a first gas pressure sensor of the pair of gas pressure sensors within an inner chamber of a first ear muff that is configured to surround a first ear of the patient. The method also comprises locating a second gas pressure sensor of the pair of gas pressure sensors within an inner chamber of a second ear muff that is configured to surround a second ear of the patient. In step b) the measurement of effectiveness of fit of the pair of ear muffs around the ears of the patient can comprise at least one measured gas pressure level.

In an example, the at least one sensor system comprises a pair of gas injectors and a pair of gas sensors, and the method comprises locating a first gas injector and a first gas sensor within an inner chamber of a first ear muff that is configured to surround a first ear of the patient. The first gas injector is configured to inject a gas other than air into the inner chamber. The method also comprises locating a second gas injector and a second gas sensor within an inner chamber of a second ear muff that is configured to surround a second ear of the patient. The second gas injector is configured to inject a gas other than air into the inner chamber. In step b) the measurement of effectiveness of fit of the pair of ear muffs around the ears of the patient can comprise at least one measured gas composition level.

In an example, step c) comprises comparing the measurement of effectiveness of fit of the pair of ear muffs around the ears of the patient with a threshold level, and controlling the actuation system to increase a level of compression of the fit of the pair of ear muffs around the ears of the patient on the basis of the comparison.

In an example, the at least one sensor system comprises highly resistive wiring.

In an example, the method comprises step d) receiving by the processing unit scan information from the medical imaging device, and comprises step e) controlling by the processing unit the actuation system to adjust the fit of the pair of ear muffs around the ears of the patient, the control comprising utilization of the scan information.

Fig. 3 shows an example of an adaptive ear protection system 400 for a medical imaging device 200. The adaptive ear protection system comprises a pair of ear plugs 410, at least one microphone 420, a pair of air tubes 430, a processing unit 440, and an output unit 450. The pair of ear plugs are configured to fit into the ears of a patient. The at least one microphone is external to the ear plugs. A first air tube of the pair of air tubes extends through a first ear plug of the pair of ear plugs and connects to the at least one microphone. A second air tube of the pair of air tubes extends through a second ear plug of the pair of ear plugs and connects to the at least one microphone. The at least one microphone is configured to provide at least one signal indicative of a measured sound level to the processing unit. The processing unit is configured to determine an effectiveness of fit of the pair of ear plugs in the ears of the patient, the determination comprising utilization of the at least one signal indicative of a measured sound level. The output unit is configured to output an indication that the ear plug fit is required to be adapted on the basis of the determined effectiveness of fit of the pair of ear plugs.

Fig. 3 relates to a specific example of an adaptive ear protection system with ear plugs for a medical imaging device that makes use of direct sound measurements. However, the proxy based techniques as described above for the ear muffs can also be applied here for the ear plugs.

Therefore, an example of an adaptive ear protection system for a medical imaging device comprises a pair of ear plugs, at least one sensor system, a processing unit, and an output unit. The pair of ear plugs are configured to fit into the ears of a patient. The at least one sensor system is configured to provide at least one signal indicative of a degree of fit of the ear plugs in the ears of the patient to the processing unit. The processing unit is configured to determine an effectiveness of fit of the pair of ear plugs in the ears of the patient, the determination comprising utilization of the at least one signal indicative of a degree of fit of the ear plugs in the ears of the patient. The output unit is configured to output an indication that the ear plug fit is required to be adapted on the basis of the determined effectiveness of fit of the pair of ear plugs.

In an example, the at least one sensor system comprises at least one photosensitive device. The at least one photosensitive device is configured to detect light leakage past a first ear plug when the ear plug is inserted into the ear of the patient. The at least one photosensitive device is configured also to detect light leakage past a second ear plug when the ear plug is inserted into the ear of the patient. The determination of the effectiveness of fit of the pair of ear plugs in the ears of the patient comprises utilization of at least one measured light level.

In an example, the sensor system comprises a pair of optical waveguides. A first optical waveguide of the pair of optical waveguides has one end located with respect to the first ear plug and a second end located to supply radiation to a photosensitive device. The first optical waveguide in combination with the photosensitive device is configured to detect light leakage past the first ear plug when the ear plug is inserted into the ear of the patient. A second optical waveguide of the pair of optical waveguides has one end located with respect to the second ear plug and a second end located to supply radiation to the photosensitive device. The second optical waveguide in combination with the photosensitive device is configured to detect light leakage past the second ear plug when the ear plug is inserted into the ear of the patient. The determination of the effectiveness of fit of the pair of ear plugs in the ears of the patient comprises utilization of at least one measured light level. Thus, in this manner a single photosensitive device such as a photodiode, used in combination with appropriately positioned waveguides such as optical fibres can be used to determine the effectiveness of fit of the ear plugs.

In an example the at least one sensor system comprises a pair of photosensitive devices. A first photosensitive device of the pair of photosensitive devices is associated with a first ear plug and is configured to detect light leakage past the ear plug when the ear plug is inserted in the ear of the patient. A second photosensitive device of the pair of photosensitive devices is associated with a second ear plug and is configured to detect light leakage past the ear plug when the ear plug is inserted in the ear of the patient. The determination of the effectiveness of fit of the pair of ear plugs in the ears of the patient comprises utilization at least one measured light level.

In an example the at least one sensor system comprises a pair of electrodes. A first electrode of the pair of electrodes is located at a periphery of a first ear plug such that when the first ear plug is inserted into a first ear of the patient the first electrode is configured to contact and/or be adjacent to skin of the patient. A second electrode of the pair of electrodes is located at a periphery of a second ear plug such that when the second ear plug is inserted into a second ear of the patient the first electrode is configured to contact and/or be adjacent to skin of the patient. The determination of the effectiveness of fit of the pair of ear plugs in the ears of the patient comprises utilization of at least one impedance, conductance, or capacitance level.

In an example two or more electrodes can be used around the periphery or seal of each ear plug, and impedance, conductance or capacitance can be measured between adjacent electrodes to determine an effectiveness or tightness of fit.

In an example, the at least one sensor system comprises a pair of gas pressure sensors. A first gas pressure sensor of the pair of gas pressure sensors is associated with a first ear plug such that it is within the ear when the first ear plug is inserted into the ear of the patient. A second gas pressure sensor of the pair of gas pressure sensors is associated with a second ear plug such that it is within the ear when the second ear plug is inserted into the ear of the patient. The determination of the effectiveness of fit of the pair of ear plugs in the ears of the patient comprises utilization of at least one measured gas pressure level.

In an example, the at least one sensor system comprises a pair of gas injectors and a pair of gas sensors. A first gas injector and a first gas sensor are associated with a first ear plug such that they are within the ear when the first ear plug is inserted in the ear of the patient. The first gas injector is configured to inject a gas other than air into an air cavity within the ear. A second gas injector and a second gas sensor are associated with a second ear plug such that they are within the ear when the second ear plug is inserted in the ear of the patient. The second gas injector is configured to inject a gas other than air into an air cavity within the ear. The determination of the effectiveness of fit of the pair of ear plugs in the ears of the patient comprises utilization of at least one measured gas composition level.

In an example, the at least one sensor system comprises highly resistive wiring.

Thus, wiring is provided that can sit for example in the MRI machine and be RF compatible.

In an example, the adaptive ear protection system is configured to operate with a magnetic resonance imaging device, scanner or imager.

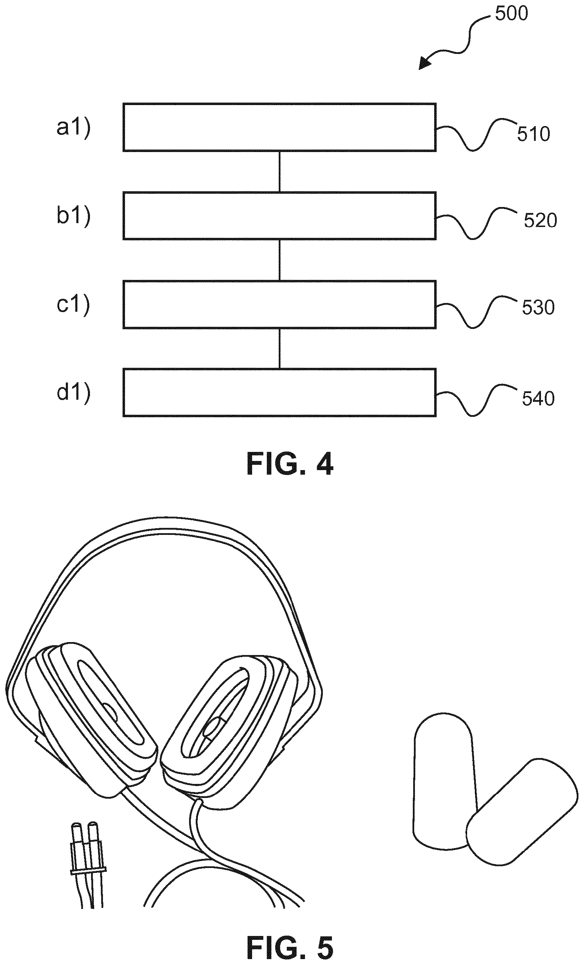

Fig. 4 shows a method 500 of adaptive ear protection for a medical imaging device in its basic steps. The method comprises:
- in a fitting step, also referred to as step a1), fitting a pair of ear plugs into the ears of a patient, wherein at least one microphone is external to the ear plugs, and wherein a first air tube of a pair of air tubes extends through a first ear plug of the pair of ear plugs and connects to the at least one microphone, and wherein a second air tube of the pair of air tubes extends through a second ear plug of the pair of ear plugs and connects to the at least one microphone;
- in a providing step 520, also referred to as step b1), providing by the at least one microphone at least one signal indicative of a measured sound level to a processing unit;
- in a determining step 530, also referred to as step c1), determining by the processing unit an effectiveness of fit of the pair of ear plugs in the ears of the patient, the determining comprising utilizing the at least one signal indicative of a measured sound level; and
- in an outputting step 540, also referred to as step d1), outputting by an output unit an indication that the ear plug fit is required to be adapted on the basis of the determined effectiveness of fit of the pair of ear plugs.

Fig. 4 relates to a specific example of an adaptive ear protection method for a medical imaging device that makes use of direct sound measurements. However, the proxy based techniques as described above for the ear muffs can also be applied here.
Therefore, an example of an adaptive ear protection method for a medical imaging device comprises:
- fitting a pair of ear plugs into the ears of a patient;
- providing by at least one sensor system at least one signal indicative of a degree of fit of the ear plugs in the ears of the patient to a processing unit;
- determining by the processing unit an effectiveness of fit of the pair of ear plugs in the ears of the patient, the determining comprising utilizing the at least one signal indicative of a degree of fit of the ear plugs in the ears of the patient; and
- outputting by an output unit an indication that the ear plug fit is required to be adapted on the basis of the determined effectiveness of fit of the pair of ear plugs.

In an example, the at least one sensor system comprises at least one photosensitive device. The method comprises detecting by at least one photosensitive device light leakage past a first ear plug when the ear plug is inserted into the ear of the patient, and the method comprises measuring a light level. The method comprises also detecting by the at least one photosensitive device light leakage past a second ear plug when the ear plug is inserted into the ear of the patient, and the method comprises measuring a light level. The determination of effectiveness of fit of the pair of ear plugs in the ears of the patient comprises utilization of at least one measured light level.

In an example the at least one sensor system comprises a pair of electrodes. A first electrode of the pair of electrodes is located at a periphery of a first ear plug such that when the first ear plug is inserted into a first ear of the patient the first electrode is configured to contact and/or be adjacent to skin of the patient, and the method comprises measuring at least one impedance, conductance, or capacitance level. A second electrode of the pair of electrodes is located at a periphery of a second ear plug such that when the second ear plug is inserted into a second ear of the patient the first electrode is configured to contact and/or be adjacent to skin of the patient, and the method comprises measuring at least one impedance, conductance, or capacitance level. The determination of the effectiveness of fit of the pair of ear plugs in the ears of the patient comprises utilization of at least one impedance, conductance, or capacitance level.

In an example two or more electrodes can be used around the periphery or seal of each ear plug, and impedance, conductance or capacitance can be measured between adjacent electrodes to determine an effectiveness or tightness of fit.

In an example, the at least one sensor system comprises a pair of gas pressure sensors. A first gas pressure sensor of the pair of gas pressure sensors is associated with a first ear plug such that it is within the ear when the first ear plug is inserted into the ear of the patient, and the method comprises measuring a gas pressure level. A second gas pressure sensor of the pair of gas pressure sensors is associated with a second ear plug such that it is within the ear when the second ear plug is inserted into the ear of the patient, and the method comprises measuring a gas pressure level. The determination of the effectiveness of fit of the pair of ear plugs in the ears of the patient comprises utilization of at least one measured gas pressure level.

In an example, the at least one sensor system comprises a pair of gas injectors and a pair of gas sensors. A first gas injector and a first gas sensor are associated with a first ear plug such that they are within the ear when the first ear plug is inserted in the ear of the patient, and the method comprises measuring a gas composition level. The first gas injector is configured to inject a gas other than air into an air cavity within the ear. A second gas injector and a second gas sensor are associated with a second ear plug such that they are within the ear when the second ear plug is inserted in the ear of the patient. The second gas injector is configured to inject a gas other than air into an air cavity within the ear, and the method comprises measuring a gas composition level. The determination of the effectiveness of fit of the pair of ear plugs in the ears of the patient comprises utilization of at least one measured gas composition level.

In an example, the at least one sensor system comprises highly resistive wiring.

Thus, wiring is provided that can sit for example in the MRI machine and be RF compatible.

In an example, the adaptive ear protection system is configured to operate with a magnetic resonance imaging device, scanner or imager.

The adaptive ear protection system for a medical imaging device and method of adaptive ear protection for a medical imaging device are now described in specific detail with respect to protection for a patient undergoing a MRI examination or scan or series of scans, where reference is made to Figs. 5-7. In specific embodiments described, an actuation system in the form of a pneumatic system is utilized, however the actuation system can be a hydraulic system or an electromagnetic system for example.

As discussed above, ear muffs and in-ear foam plugs can be used to attenuate sound for a patient undergoing MRI examination, but there are failings, including an inability to monitor the attenuation level provided, and an inability to ensure that the correct level of attenuation is being provided.

The adaptive ear protection system for a medical imaging device and method of adaptive ear protection for a medical imaging device described here can be designed in a number of specific ways to address this. Thus, one mechanism is to measure the effectiveness of the ear protection devices, such as ear muffs, by additional microphones. Electrical wiring of the ear muffs should be avoided in the MRI device, for RF safety reasons. Therefore, air tubes are used to provide a connection between the ear muffs and the microphones. The microphones are then integrated into or with the in-room operating console of the MRI device or system, with the MR head coil, or with the patient support, so that the wired microphones are at a safe distance from the patient. The air tubes are safe in all MR environments (Set-up / MR field-strengths / MR sequences), and the air tubes can also be used to communicate with the patient via loudspeakers that are external to the ear muffs. The electrical loudspeakers need low resistance wiring, which is prohibitive in MR, and thus the air tubes are used to communicate with the patient and also used to determine an effectiveness of fit of the ear muffs by relaying sound from within the ear muffs to microphones external to the ear muffs. In this way, the air tubes can be used for both purposes.

The ear muffs are provided with a pneumatic system to adjust the compression to the head of the patient. A control system then measures the sound level with above microphones during scanning, and if required adapts the compression of the ear muffs to achieve a comfortably low, but sufficient, level of compression. However, rather than directly measure the sound level another mechanism is to assess the effectiveness of the ear protection devices, by what can be termed a "proxy" method. The proxy method involves measuring another physical parameter (light level, electrical signal,) behind the ear muff protection (on the side of ear) or alternatively at the interface between the ear muff protection and the head of the patient. This measured physical parameter then relates to the acoustic effectiveness of the attenuation being provided. As such the proxy method also enables an automatic check that ear protection is still in place by monitoring these proxy physical signal changes. Using either approach, the overall system may ask the patient manually to adjust the devices in situation when the system measures that insufficient protection is being provided. The system may also finally alert the radiographer if this does not help, but could do so external to any patient notification as well. The system can also increase the compression during loud scans and release compression somewhat during silent periods to increase comfort. If the compression is increased a few seconds before a loud scan section begins, the user is intuitively warned to prepare for the loud section, which may reduce anxiety. The system can completely release ear muffs if the exam is over.

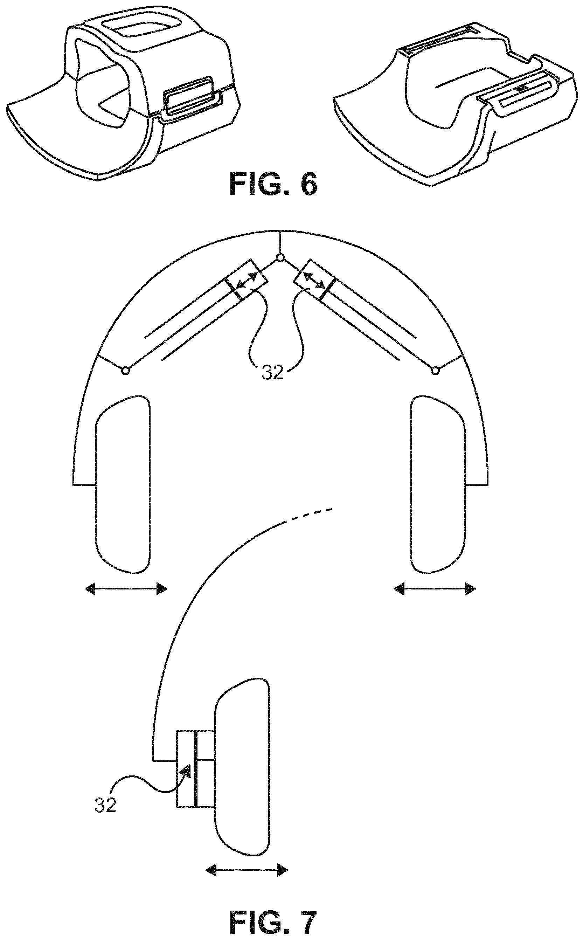

The ear muffs, similar to those shown in Fig. 5 but with pistons as part of a pneumatic system to change the level of compression provided around the head, are equipped with twin air tubes for use during an MRI scan or examination. The air tubes extend into the ear muff and have an opening on the ear side of the ear muffs, where sound is being attenuated. The air tubes are connected to loudspeakers in the MRI system to provide music or operator instructions to the patient. However, the air tunes are additionally connected to a pair of microphones in a part of the MR system, that separately measures the sound levels at both ears of the patient within the ear muffs. If appropriate high resistance circuitry is provided the microphones can be positioned within the ear muffs, in the attenuated region, but as the air tubes are provided for communication with the patient it has been found to be easier to use standard microphones that are external to the RF active part of the MRI device, and use those air tubes to relay sound to the microphones and this has been found to be a particularly advantageous embodiment. The microphones can be integrated into the communication unit of the MRI system that holds the loudspeakers used in connection with the MR ear muffs displayed. This can require long air tubes, which may introduce additional noise into the microphone system when moving the tubes. Therefore, the microphones can alternatively be integrated into the MR head coil (See Fig. 6). The air tubes coming from the ear muffs are connected by the staff to hose adapters integrated in the MR headcoil, which internally holds the microphones. The microphones may similarly be integrated with the patient support if necessary. The air tubes can actually extend into the ear muffs, and then extend through ear plugs (similar to those shown in Fig. 5), where the patient has the ear plugs inserted into their ears. In this way, the sound level, indicative of an effectiveness of fit is measured at exactly the right position, adjacent to the ear canal. Indeed, the system can work without the ear muffs. Here. The air tunes extend through the ear plugs and to microphones, and sound levels picked up are sent to a processing unit that provides an output if the sound level is too great. Thus, even if the patient wears only ear plugs without the ear muffs, an adaptive reaction to a too high in-ear sound level can be provided by issuing warnings and triggering a patient/ staff action, or even by providing an automatic scan abort.

Fig. 7 shows the ear muffs, equipped with a pneumatic compression system, fed by pressurized air provided through an additional lumen in the connection tube. Thus, the connection tube or air tube is a tube with two lumina and as such is essential a compound tube consisting of two tubes. One tube is for the sound communication, and a second tube is for the pneumatic system. A software-controlled piston integrated into the MR system connects to the second tube for the pneumatic system that connects to a second set of pistons in the ear muff.

Then, two pistons of the pneumatic compression system can be utilized with the main structure of the ear muffs, where increased air pressure decreases the compression of the muffs by pushing the two ear muffs away from each other, as shown in Fig. 5 where extension of the two pistons moves the ear muffs away from each other. Additionally, an additional piston associated with an ear muff can be used to move the ear muff away from the main structure in an inward direction, to compress both ear muffs against the patients head, with increased air pressure used to increases the compression of the muffs. This is shown in Fig. 7 with respect to the single ear muff shown, where the piston is adjacent to the ear muff. Thus, for compression there need only be one piston associated with one ear muff to compress both ear muffs. However, more complex relaxation and compression systems can be provided where the ear muffs can move independently due to a part of the main structure of the ear muffs being immobile with respect to the patient's head for example. Thus, the clamp or main structure of the MR ear muffs is provided with an air-pressure-actuated system to adjust the compression to the head of the patient. This avoids any wiring and active electrical devices that may interfere with the MR equipment, maintaining MR safety and image quality during scanning. Multiple embodiments are possible to realize the mechanics of the pneumatic system, as described above. They all have in common that a lumen in a tube of the ear muff provides pressurized air to some form of actuator in the clamp. The pressurized air is provided by a software-controlled piston integrated in the MR system. The actuator associated with the second set of pistons can work against springs, thereby either releasing the ear muffs from the head or compressing them to the head of the patient.

It was established that it is not always necessary to measure the sound level at the ear to assess the effectiveness of the ear protection, and this led to the development of the above discussed proxy methods, which are described in more detail below. Specifically, it has been established that the effectiveness of the acoustic protection provided by the ear muffs can be related to the quality of the seal between the ear muffs and the skin surrounding the patient's ear. The monitoring of this level of seal led to the different proxy methods to determine an effectiveness of fit that could be related to the acoustic attenuation.

Thus, as now described a series of proxy measurements can be used to assess the quality of this seal between the ear muffs and the patients and hence - indirectly - the effectiveness of the ear protection provided by the ear muffs. The proxy methods can take any of the following physical measurement approaches:
A light based approach in which the quality of the seal is assessed using a light leakage approach across the seal. For example in a simple approach a photosensitive device (like a photodiode) is added to the inside of the ear muff - which is ideally non-transparent. Any light leaking from the environment into the ear muff will be detected by the photosensor and is a direct indication of a poor seal - which in turn will reduce the effectiveness of the protection

In a first electrical approach, two electrodes are added at the seal position to make contact with the patient's skin and the impedance / conductance between the electrodes is measured. The conductance / capacitance increases with the firmness of the contact of the electrodes with the skin, which can be used as an indication of good seal after a suitable calibration. Highly resistive wiring is used for the electrical set-up to ensure RF safety for this embodiment.

In a second electrical approach in which the quality of the seal is assessed using an electrical signal at the seal. A single thin film electrode is added close to the seal position, isolated from the patient's skin by a thin insulating layer. A measurement is then made of the capacitance against a common ground plane, i.e. the RF screen of the body coil. A reduction in capacitance results when the spacing of the electrode from the skin increases and is a direct indication of a poor seal - which in turn reduces the effectiveness of the protection. Highly resistive wiring is again used for the electrical set-up to ensure RF safety for this embodiment.

Further suitable electrical set-ups using one or more electrodes and measurement of mutual complex impedances between those maybe used to determine whether ear protection devices (ear muffs, ear plugs) provide a tight seal.

Another approach is based on a slight constant positive or negative air pressure within the ear muff. Any loss of tight fit will result in a pressure drop, or if a feedback loop is used to keep the pressure constant, into an increase of air supply to keep the pressure at preset level.

Because the ear is very susceptible to pressure differences, another approach is to use a different gas than air (nitrogen, carbon dioxide, argon) within the muffs at surrounding pressure and to measure the gas composition within the muff. Any ingress of air indicates a loss of a tight seal. Gas sensors are relatively inexpensive and can be selected such that they can be operated with highly resistive wiring to ensure RF safety.

Several of these approaches have advantages in the MR compatibility and simplicity of the physical principle compared to the direct measurement of the sound level.

In all of the above approaches, the measured effectiveness of fit of the ear muffs, directly determined from a sound level, or using the above proxy methods, can be used with the pneumatic (or other actuation) system to adjust the compression of the ear muffs to the patient's head to provide the correct, and comfortable, level of protection.

A control system or processing unit can measure the sound level with the above described microphones (or via one of the proxy methods) during scanning and compares it to a threshold that provides a safe sound level. If the sound level (either measured directly or determined from a level of the seal of the ear muffs from a proxy method in association with a calibration factor) is above the threshold, the system increases the compression of the ear muffs to achieve a better sealing of the ear muffs. Otherwise, the compression is released to a more comfortable level. The system can ask the patient manually to adjust the devices in the situation where the system measures insufficient protection even for maximum compression. The system may finally alert the radiographer if this does not help. The system can increase the compression during loud scans and release compression during silent periods to increase overall comfort. The control system can also increase the compression if it is determined from a proxy method that the ear muff seal is not tight enough, and need not use a threshold. Thus, for example once the ear muff is tight after compression, for example light tightness is provided, a further degree of tightness can be provided via further compression.

The above described adaptive ear protection systems and associated methods of ear protection have certain advantages, including that the methods avoid the use of a listening test to test the effectiveness of ear protection, which is subjective, costs time, and may not be performed consistently, e.g. due to time pressure or inability of the patient to comply. The method allows permanent control during the entire exam and immediate action to be undertaken. The method catches cases where patients remove the ear muff (or ear plugs) protection during scanning, or where the attenuation is lowering during the scan because of loss of tight fit of the devices.

### High Resistance Circuitry

Reference is made above to high resistance circuitry, used for example in the above described proxy methods. These electrical embodiments can involve the measurement of some resistance across a certain part of the skin, which is typically in the range of 0.1-10MOhm. Thus, the high resistance circuitry can relate to small skin patches and attached resistive wires with about 10kOhm/m leading to a pre-amplifier several tens of cm away from the patient, which can be used safely and without affecting the resistance measurement. Such circuitry has been shown to be safe in MR under defined.

In another exemplary embodiment, a computer program or computer program element is provided that is characterized by being configured to execute the method steps of the method according to one of the preceding embodiments, on an appropriate system.

The computer program element might therefore be stored on a computer unit such as a smartphone, laptop, tablet, or a computer unit within an oral cleaning device such as a toothbrush, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An adaptive ear protection system (10) for a medical imaging device (200), the adaptive ear protection system comprising:
- a pair of ear muffs (20);
- an actuation system (30);
- at least one sensor system (40); and
- a processing unit (50);
wherein, the pair of ear muffs are configured to fit around the ears of a patient;
wherein, the actuation system is configured to adjust the fit of the pair of ear muffs around the ears of the patient;
wherein, the at least one sensor system is configured to determine a measurement of an effectiveness of fit of the pair of ear muffs around the ears of the patient; and
wherein, the processing unit is configured to control the actuation system to adjust the fit of the pair of ear muffs around the ears of the patient, the control comprising utilization of the measurement of effectiveness of fit of the pair of ear muffs around the ears of the patient.

2. Adaptive ear protection system according to claim 1, wherein the sensor system is configured to provide the at least one measurement of effectiveness of fit of the pair of ear muffs around the ears of the patient to the patient; wherein the processing unit is configured to receive an ear muff fit instruction; and wherein the processing unit is configured to control the actuation system to adjust the fit of the pair of ear muffs around the ears of the patient comprising utilization of the ear muff fit instruction.

3. Adaptive ear protection system according to any of claims 1-2, wherein the at least one sensor system comprises at least one microphone (60); and wherein the measurement of effectiveness of fit of the pair of ear muffs around the ears of the patient comprises at least one measured sound level.

4. Adaptive ear protection system according to claim 3, wherein the at least one microphone is external to the pair of ear muffs.

5. Adaptive ear protection system according to claim 4, wherein the at least one sensor system comprises a pair of air tubes (62), wherein a first air tube of the pair of air tubes connects an inner chamber of a first ear muff that is configured to surround a first ear of the patient to the at least one microphone, and wherein a second air tube of the pair of air tubes connects an inner chamber of a second ear muff that is configured to surround a second ear of the patient to the at least one microphone.

6. Adaptive ear protection system according to any of claims 1-2, wherein the at least one sensor system comprises at least one photosensitive device (70), wherein the at least one photosensitive device is configured to detect light leakage past a seal around an inner chamber of a first ear muff that is configured to surround a first ear of the patient, wherein the at least one photosensitive device is configured to detect light leakage past a seal around an inner chamber of a second ear muff that is configured to surround a second ear of the patient, and wherein the measurement of effectiveness of fit of the pair of ear muffs around the ears of the patient comprises at least one measured light level.

7. Adaptive ear protection system according to any of claims 1-2, wherein the at least one sensor system comprises a pair of electrodes (80), wherein a first electrode of the pair of electrodes is located at a periphery of a first ear muff such that when the first ear muff is positioned to surround a first ear of the patient the first electrode is configured to contact and/or be adjacent to skin of the patient and a second electrode of the pair of electrodes is located at a periphery of a second ear muff such that when the second ear muff is positioned to surround a second ear of the patient the second electrode is configured to contact and/or be adjacent to skin of the patient; and wherein the measurement of effectiveness of fit of the pair of ear muffs around the ears of the patient comprises at least one impedance, conductance, or capacitance level.

8. Adaptive ear protection system according to any of claims 1-2, wherein the at least one sensor system comprises a pair of gas pressure sensors (90), wherein a first gas pressure sensor of the pair of gas pressure sensors is located within an inner chamber of a first ear muff that is configured to surround a first ear of the patient and a second gas pressure sensor of the pair of gas pressure sensors is located within an inner chamber of a second ear muff that is configured to surround a second ear of the patient; and wherein the measurement of effectiveness of fit of the pair of ear muffs around the ears of the patient comprises at least one measured gas pressure level.

9. Adaptive ear protection system according to any of claims 1-2, wherein the at least one sensor system comprises a pair of gas injectors (100) and a pair of gas sensors (110), wherein a first gas injector and a first gas sensor is located within an inner chamber of a first ear muff that is configured to surround a first ear of the patient, wherein the first gas injector is configured to inject a gas other than air into the inner chamber; and wherein a second gas injector and a second gas sensor is located within an inner chamber of a second ear muff that is configured to surround a second ear of the patient, wherein the second gas injector is configured to inject a gas other than air into the inner chamber; and wherein the measurement of effectiveness of fit of the pair of ear muffs around the ears of the patient comprises at least one measured gas composition level.

10. Adaptive ear protection system according to any of claims 1-9, wherein utilization of the measurement of effectiveness of fit of the pair of ear muffs around the ears of the patient comprises a comparison of the measurement of effectiveness of fit of the pair of ear muffs around the ears of the patient with a threshold level, and wherein the processing unit is configured to control the actuation system to increase a level of compression of the fit of the pair of ear muffs around the ears of the patient on the basis of the comparison.

11. Adaptive ear protection system according to any of claims 1-10, wherein the processing unit is configured to receive scan information from the medical imaging device (200), and wherein the processing unit is configured to control the actuation system to adjust the fit of the pair of ear muffs around the ears of the patient, the control comprising utilization of the scan information.

12. An adaptive ear protection system (400) for a medical imaging device (200), the adaptive ear protection system comprising:
- a pair of ear plugs;
- at least one sensor system;
- a processing unit; and
- an output unit;
wherein, the pair of ear plugs are configured to fit into the ears of a patient;
wherein, the at least one sensor system is configured to provide at least one signal indicative of a degree of fit of the ear plugs in the ears of the patient to the processing unit;
wherein, the processing unit is configured to determine an effectiveness of fit of the pair of ear plugs in the ears of the patient, the determination comprising utilization of the at least one signal indicative of a degree of fit of the ear plugs in the ears of the patient; and
wherein, the output unit is configured to output an indication that the ear plug fit is required to be adapted on the basis of the determined effectiveness of fit of the pair of ear plugs

13. A method (300) of adaptive ear protection for a medical imaging device, the method comprising:
a) fitting (310) a pair of ear muffs the ears of a patient, wherein an actuation system is configured to adjust the fit of the pair of ear muffs around the ears of the patient;
b) determining (320) by at least one sensor system a measurement of an effectiveness of fit of the pair of ear muffs around the ears of the patient; and
c) controlling (330) by a processing unit the actuation system to adjust the fit of the pair of ear muffs around the ears of the patient, the controlling comprising utilizing the measurement of effectiveness of fit of the pair of ear muffs around the ears of the patient.

14. A method (500) of adaptive ear protection system for a medical imaging device, the method comprising:
- fitting a pair of ear plugs into the ears of a patient;
- providing by at least one sensor system at least one signal indicative of a degree of fit of the ear plugs in the ears of the patient to a processing unit;
- determining by the processing unit an effectiveness of fit of the pair of ear plugs in the ears of the patient, the determining comprising utilizing the at least one signal indicative of a degree of fit of the ear plugs in the ears of the patient; and
- outputting by an output unit an indication that the ear plug fit is required to be adapted on the basis of the determined effectiveness of fit of the pair of ear plugs.

15. A computer program element for controlling a system according to any of claims 1 to 11, which when executed by a processor is configured to carry out the method of claim 13, and/or for controlling a system according to claim 12, which when executed by a processor is configured to carry out the method of claim 14.
